# EUROPEAN PATENT APPLICATION

(11) **EP 3 777 951 A1**
(43) Date of publication of application: **17.02.2021**
(21) Application number: 19774264.6
(22) Date of filing: 29.03.2019
(51) Int. Cl.: A61M 25/09, A61B 5/0215

(54) **GUIDE WIRE**

(30) Priority: 30.03.2018 US 201862650909 P
(71) Applicant: ASAHI INTECC CO., LTD., Seto-shi, Aichi 489-0071 (JP)
(72) Inventor: SAWAI Akira, Seto-shi, Aichi 489-0071 (JP); SUGITA Shuji, Seto-shi, Aichi 489-0071 (JP)
(74) Representative: Winter, Brandl, Fürniss, Hübner, Röss, Kaiser, Polte - Partnerschaft mbB
(86) International application number: PCT/JP2019/014185
(87) International publication number: WO 2019/189828

(57) **Abstract**

Provided is a guide wire including a detection portion, an accommodating portion, and a covering portion. The detection portion has a detection element for detecting information about a vessel in an inside of the vessel, and an extension portion for transmitting the information detected with the detection element, the extension portion extending from the detection element toward a proximal end side of the guide wire. The accommodating portion has a first accommodating portion accommodating the detection element and a second accommodating portion accommodating the extension portion. The covering portion covers the second accommodating portion. A distal end portion of the covering portion is connected to a proximal end portion of the first accommodating portion.

## Description

### TECHNICAL FIELD

The present invention relates to a guide wire.

### BACKGROUND ART

Conventionally, a guide wire is known which is to be inserted into a blood vessel to detect (measure) intravascular pressure (hereinafter, referred to as "blood pressure"). For example, Patent Document 1 discloses a pressure-sensing guide wire including a pressure sensor disposed inside a tubular member. In such a pressure-sensing guide wire, a pressure sensor for detecting blood pressure needs to be brought into contact with blood. Accordingly, in Patent Document 1, a plurality of slots are formed in the tubular member for allowing blood to flow into the inside of the tubular member. For example, Patent Document 2 discloses a tubular member (a medical tube) having circular slots formed in a spiral manner or with a predetermined spacing.

### CITATION LIST

### Patent Document

Patent Document 1: Japanese Translation of PCT International Application Publication No. 2016-510679
Patent Document 2: Japanese Patent Application Laid-Open No. H8-257128

### DISCLOSURE OF THE INVENTION

### PROBLEMS TO BE SOLVED BY THE INVENTION

Here, a guide wire is assumed to have a small diameter so as to be inserted into a blood vessel, and the blood vessel is assumed to be curved in a complex manner. Under these circumstances, the pressure-sensing guide wire described in Patent Document 1 or the guide wire including a medical tube described in Patent Document 2, both of which have a plurality of slots formed circumferentially, may suffer from the risk of scattering of the tubular member during use of the guide wires due to stress concentrated at a position between one slot and another. A scattered piece which would be generated if the tubular member is scattered may damage body tissues, and also needs to be removed with laborious efforts if it remains inside the body. This is not preferred. Further, the medical tube having spirally arranged slots as described in Patent Document 2 may suffer from a problem in that the torquability is poor when operated rotationally in a direction opposite to the winding direction of the spirally arranged slots.

It is noted that these problems are shared with not only a guide wire to be inserted into a blood vessel for detecting blood pressure but also a guide to be inserted into a vessel for detecting/obtaining information (for example, pressure, temperature, an image, and the like) about the vessel. These problems are also shared with guide wires to be inserted not only into the blood vessel system but also into organs of the human body such as the lymph gland system, the biliary tract system, the urinary tract system, the respiratory tract system, the digestive organ system, secretory glands, reproductive organs, and the like.

The present invention is made in order to solve at least partially the aforementioned problems. An object of the present invention is to reduce the risk of scattering for a guide wire capable of obtaining information about a vessel in the inside of the vessel.

### MEANS FOR SOLVING THE PROBLEMS

The present invention is made in order to solve at least partially the aforementioned problems, and can be implemented in the following forms.
(1) According to an aspect of the present invention, a guide wire is provided. The above guide wire includes: a detection portion having a detection element for detecting information about a vessel in an inside of the vessel and an extension portion for transmitting the information detected with the detection element, the extension portion extending from the detection element toward a proximal end side of the guide wire; an accommodating portion having a first accommodating portion accommodating the detection element and a second accommodating portion accommodating the extension portion; and a covering portion covering the second accommodating portion, the covering portion having a distal end portion connected to a proximal end portion of the first accommodating portion.
   In general, the extension portion for transmitting the information detected with the detection element has a smaller diameter than the detection element for detecting information about a vessel. According to the above configuration where the covering portion is included which further covers the second accommodating portion accommodating the extension portion having a small diameter, the presence of the covering portion can prevent scattering of the guide wire, and can also prevent a scattering piece from remaining in the body even if breakage occurs in the second accommodating portion. Further, the distal end portion of the above covering portion is connected to the proximal end portion of the first accommodating portion accommodating the detection element. This configuration can prevent scattering of the guide wire at the boundary between the first accommodating portion and the second accommodating portion, and can also prevent a scattered piece from remaining in the body. Moreover, the above configuration where the distal end portion of the covering portion is connected to the proximal end portion of the first accommodating portion can improve the torquability when the guide wire is rotationally operated. These features of the guide wire according to the present aspect can reduce the risk of scattering for the guide wire capable of information about a vessel in the inside of the vessel.
(2) In the guide wire according to the above aspect, the covering portion may have an outer diameter at the distal end portion substantially equal to an outer diameter of the proximal end portion of the first accommodating portion, and may be arranged side by side coaxially with the first accommodating portion. According to the above configuration, the covering portion has an outer diameter at the distal end portion substantially equal to that at the proximal end portion of the first accommodating portion, and is arranged side by side substantially coaxially with the first accommodating portion. This configuration can allow an outer surface of the guide wire at a connection section between the distal end portion of the covering portion and the proximal end portion of the first accommodating portion to be shaped to be flat without unevenness. This in turn can prevent damages to tissues inside a vessel.
(3) In the guide wire according to the above aspects, a diameter-decreasing portion having an outer diameter gradually decreasing from a proximal end side to a distal end side may be formed at a distal end side of the second accommodating portion. The above configuration where the diameter-decreasing portion having an outer diameter gradually decreasing from the proximal end side to the distal end side is formed at the distal end side of the second accommodating portion enables the distal end side of the second accommodating portion to be configured to be flexible as compared with the proximal end side of the second accommodating portion.
(4) In the guide wire according to the above aspects, a stepped portion engaging with the distal end portion of the covering portion may be formed at the proximal end portion of the first accommodating portion. The above configuration where the stepped portion engaging with the distal end portion of the covering portion is formed at the proximal end portion of the first accommodating portion enables easy positioning of the first accommodating portion with the covering portion at the time of manufacture. Further, locally increased stiffness can be avoided at the connection section between the first accommodating portion and the covering portion as compared with a configuration where a stepped portion is not formed.
(5) In the guide according to the above aspects, the followings may be included: a shaft portion disposed on a proximal end side of the second accommodating portion; and a proximal end side-covering portion covering a part of the proximal end side of the second accommodating portion not covered with the covering portion, a boundary portion between the second accommodating portion and the shaft portion, and a part of a distal end side of the shaft portion. A second region may have a stiffness equal to or larger than a stiffness of a first region, the first region being a region of the second accommodating portion covered with the covering portion but not covered with the proximal end side-covering portion, and the second region being a region of the second accommodating portion not covered with the covering portion but covered with the proximal end side-covering portion. The above configuration enables the stiffness of the guide wire to be gradually decreased from the proximal end side to the distal end side thereof, and can provide a guide wire excellent in supporting capability, torquability, and vascular selectivity. Further, the above configuration where the proximal end side-covering portion covers the boundary portion between the second accommodating portion and the shaft portion and the both ends thereof (that is, a part of the proximal end side of the second accommodating portion not covered with the covering portion and a part of the distal end portion of the shaft portion) can prevent occurrence of a breakage and/or a kink at the boundary portion between the second accommodating portion and the shaft portion, leading to a guide wire having improved durability.
(6) In the guide wire according to the above aspects, a third region may have a stiffness equal to or larger than a stiffness of the second region, the third region being a region of the shaft portion covered with the proximal end side-covering portion. The above configuration enables the stiffness of the guide wire to be gradually decreased from the proximal end side to the distal end side thereof, and can provide a guide wire excellent in supporting capability, torquability, and vascular selectivity.
(7) In the guide wire according to the above aspects, a fourth region may have a stiffness equal to or larger than a stiffness of the third region, the fourth region being a region of the shaft portion not covered with the proximal end side-covering portion. The above configurations enables the stiffness of the guide wire to be gradually decreased from the proximal end side to the distal end side thereof, and can provide a guide wire excellent in supporting capability, torquability, and vascular selectivity.
(8) In the guide wire according to the above aspects, the first accommodating portion, the second accommodating portion, and the proximal end side-covering portion may be formed of a hyperelastic material, and the covering portion and the shaft portion may be formed of a material more plastically deformable than the hyperelastic material. When a hyperelastic material having high fatigue strength is used in the above configuration, the fracture durability of the first accommodating portion, the second accommodating portion, and the proximal end side-covering portion can be improved. Further, the above configuration where the material more plastically deformable than the hyperelastic material is used for the covering portion and the shaft portion enables the stiffness of the guide wire to be gradually decreased from the proximal end side to the distal end side thereof, and can provide a guide wire excellent in supporting capability, torquability, and vascular selectivity.
(9) In the guide wire according to the above aspects, the first accommodating portion and the second accommodating portion may be formed of a hyperelastic material, and the covering portion, the proximal end side-covering portion, and the shaft portion may be formed of a material more plastically deformable than the hyperelastic material. When a hyperelastic material having high fatigue strength is used in the above configuration, the fracture durability of the first accommodating portion and the second accommodating portion can be improved. Further, the above configuration where the material more plastically deformable than the hyperelastic material is used for the covering portion, the proximal end side-covering portion, and the shaft portion enables the stiffness of the guide wire to be gradually decreased from the proximal end side to the distal end side thereof, and can provide a guide wire excellent in supporting capability, torquability, and vascular selectivity.
(10) In the guide wire according to the above aspects, the covering portion may be a coil body configured such that one or more element wires are wound spirally. In the above configuration where the covering portion is a coil body configured such that one of more element wires are spirally wound, breakage at both the covering portion and the second accommodating portion, if occurs, would merely cause the element wire(s) of the covering portion to unwind and extend. This can further prevent scattering of the guide wire, and can further prevent a scattered piece from remaining in the body.
(11) In the guide wire according to the above aspects, the detection element may be for detecting a pressure of a body fluid flowing through the inside of the vessel. In the above configuration where the detection element is for detecting the pressure of a body fluid flowing through the inside of the vessel, the guide wire can serve as a device for detecting (measuring) blood pressure.
(12) In the guide wire according to the above aspects, a distal end coil may be disposed on a distal end side of the first accommodating portion, the distal end coil being configured such that one or more element wires are wound spirally. The presence of the distal end coil disposed on the distal end side of the first accommodating portion and configured such that one or more element wires are wound spirally can improve the flexibility at the distal end side, reducing the risk of damages to tissues inside the vessel.

It is noted that the present invention can be implemented according to various aspects, for example, according to the forms of a guide wire capable of obtaining information about a vessel in the inside of the vessel, a method of manufacturing the guide wire, a device for manufacturing the guide wire, and the like.

### BRIEF DESCRIPTION OF THE DRAWINGS

Fig. 1 shows an illustrative diagram of a cross-sectional configuration of a guide wire according to a first embodiment.
Fig. 2 shows an illustrative diagram of a cross-sectional configuration along the A-A line in Fig. 1.
Fig. 3 shows an illustrative diagram of the configuration of an intermediate portion.
Fig. 4 shows an illustrative diagram of the configuration of a proximal end portion.
Fig. 5 shows an illustrative diagram of the configuration of an intermediate portion of a guide wire according to a second embodiment.
Fig. 6 shows an illustrative diagram of the configuration of a proximal end portion of the guide wire according to the second embodiment.
Fig. 7 shows an illustrative diagram of the configuration of an intermediate portion of a guide wire according to a third embodiment.
Fig. 8 shows an illustrative diagram of the configuration of an intermediate portion of a guide wire according to a fourth embodiment.
Fig. 9 shows an illustrative diagram of the configuration of a proximal end portion of a guide wire according to a fifth embodiment.
Fig. 10 shows an illustrative diagram of the configuration of an intermediate portion of a guide wire according to a sixth embodiment.
Fig. 11 shows an illustrative diagram of a cross-sectional configuration of a guide wire according to a seventh embodiment.
Fig. 12 shows an illustrative diagram of a cross-sectional configuration of a guide wire according to an eighth embodiment.
Fig. 13 shows an illustrative diagram of the configuration of the intermediate portion of a guide wire according to a ninth embodiment.

### PREFERRED MODE FOR CARRYING OUT THE INVENTION

### First embodiment

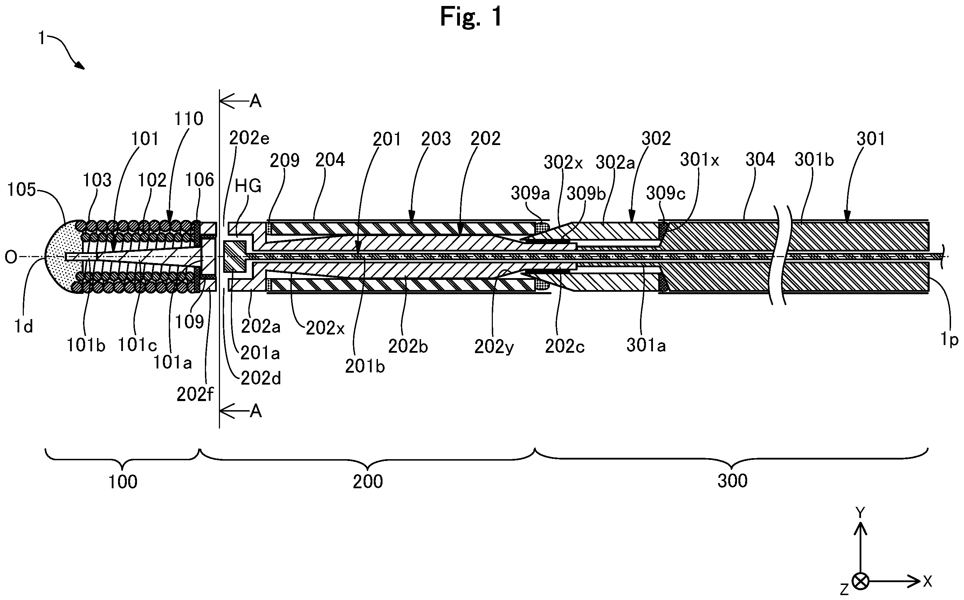

Fig. 1 shows an illustrative diagram of a cross-sectional configuration of a guide wire 1 according to a first embodiment. The guide wire 1 according to the present embodiment is a device which is to be inserted into a blood vessel, and can be used to detect blood pressure (intravascular pressure). The blood pressure detected with the guide wire 1 can be used to derive a coronary fractional flow reserve (FFR). The coronary fractional flow reserve, which represents a pressure after a stenotic segment relative to a pressure before the stenotic segment, is used as a measure of the physiological severity of stenosis.

In Fig. 1, an axis line O (a dot-and-dash line) represents an axis passing through the center of the guide wire 1. The following descriptions assume that the axis passing through the center of the guide wire 1 and an axis passing through the center of each of the members of the guide wire 1 coincide with the axis line O, but they each may be different. It is noted that the XYZ axes intersecting orthogonally to each other are shown in Fig. 1. The X-axis corresponds to the axis direction of the guide wire 1 (the insertion direction of the guide wire 1), and the Y-axis corresponds to the height direction of the guide wire 1, and the Z-axis corresponds the width direction of the guide wire 1. The left side (the -X axis direction) in Fig. 1 is referred to a "distal end side" of the guide wire 1 and constituent members thereof, and the right side (the +X axis direction) in Fig. 1 is referred to as a "proximal end side" of the guide wire 1 and constituent members thereof. Further, with regard to the guide wire 1 and constituent members thereof, an end portion and the vicinity thereof located at the distal end side is referred to an "distal end portion" or simply a "distal end," and an end portion and the vicinity thereof located at the proximal end side is referred to an "proximal end portion" or simply a "proximal end." The distal end side is to be inserted into the inside of a body, and the proximal end side is to be operated by an operator such as a physician. These also apply to the figures other than Fig. 1.

As shown in Fig. 1, the guide wire 1 includes a distal end portion 100, an intermediate portion 200, and a proximal end portion 300. The distal end portion 100 is disposed at the distal end side of the guide wire 1, and includes a distal end core 101, a distal end tip 105, and a distal end coil 110.

The distal end core 101 is a solid member disposed along the axis line O of the guide wire 1 and having an outer diameter decreasing from the proximal end side to the distal end side thereof. The distal end core 101 has a distal end portion 101b disposed at the distal end side thereof, a flange portion 101a disposed at the proximal end side thereof, a tapered portion 101c disposed between the distal end portion 101b and the flange portion 101a. The distal end portion 101b is a member with a substantially cylindrical shape having a substantially constant outer diameter, and a region from the distal end portion 101b through the middle of the tapered portion 101c has been subjected to an annealing process (annealed). The flange portion 101a is a member with a substantially cylindrical shape having a substantially constant outer diameter larger than that of the distal end portion 101b. The tapered portion 101c is a member having an outer diameter smaller than that of the flange portion 101a at the proximal end side thereof and gradually decreasing from the proximal end side to the distal end side thereof. The distal end core 101 is formed of a hyperelastic material, for example, a NiTi (nickel-titanium) alloy or an alloy of NiTi with an additional metal(s).

The distal end coil 110 is disposed so as to circumferentially surround the distal end core 101, and has a substantially constant outer diameter from the proximal end side through the distal end side thereof. The distal end coil 110 has a first coil body 102 disposed inside and a second coil body 103 disposed outside. The first coil body 102 is a single-thread twisted wire coil configured such that a plurality of element wires are twisted to be single-threaded. The second coil body 103 is a single-thread coil configured such that a single element wire is wound to be single-threaded. It is noted that the first coil body 102 and the second coil body 103 each may be a single-thread coil configured such that a single element wire is wound to be single-threaded, or may be a multi-thread coil configured such that a plurality of element wires are wound to be multi-threaded, or may be a single-thread twisted wire coil configured such that a twisted wire in which a plurality of element wires are twisted is wound to be single-threaded, or may be a multi-thread twisted wire coil configured such that a plurality of twisted wires in which a plurality of element wires are twisted are each wound to be multi-threaded. It is noted that the first coil body 102 and the second coil body 103 constitute the "distal end coil."

The proximal end portion of the first coil body 102 and the proximal end portion of the second coil body 103 are each joined to the proximal end portion of the tapered portion 101c of the distal end core 101 through a joining region 106. Joining can be achieved by using any joining agent, for example, a metal solder such as silver solder, gold solder, zinc, an Sn-Ag alloy, and an Au-Sn alloy; and an adhesive such as an epoxy-based adhesive. In the example as shown in Fig. 1, an element wire of the second coil body 103 has a larger wire diameter than an element wire of the first coil body 102. However, they may have the substantially same wire diameter, or the element wire of the second coil body 103 may have a smaller wire diameter than the element wire of the first coil body 102. The first coil body 102 and the second coil body 103 may be formed of, for example, a material more plastically deformable than a hyperelastic material, for example, a stainless steel alloy such as SUS304 and SUS316.

The distal end tip 105 is disposed at the distal end portion 100 of the guide wire 1, and joins and holds the distal end portion of the distal end core 101, the distal end portion of the first coil body 102, and the distal end portion of the second coil body 103 together. The distal end tip 105 can be formed of any joining agent, for example, a metal solder such as silver solder, gold solder, zinc, an Sn-Ag alloy, and an Au-Sn alloy; and an adhesive such as an epoxy-based adhesive.

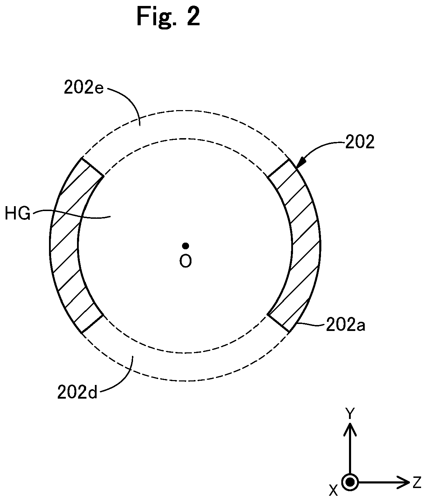

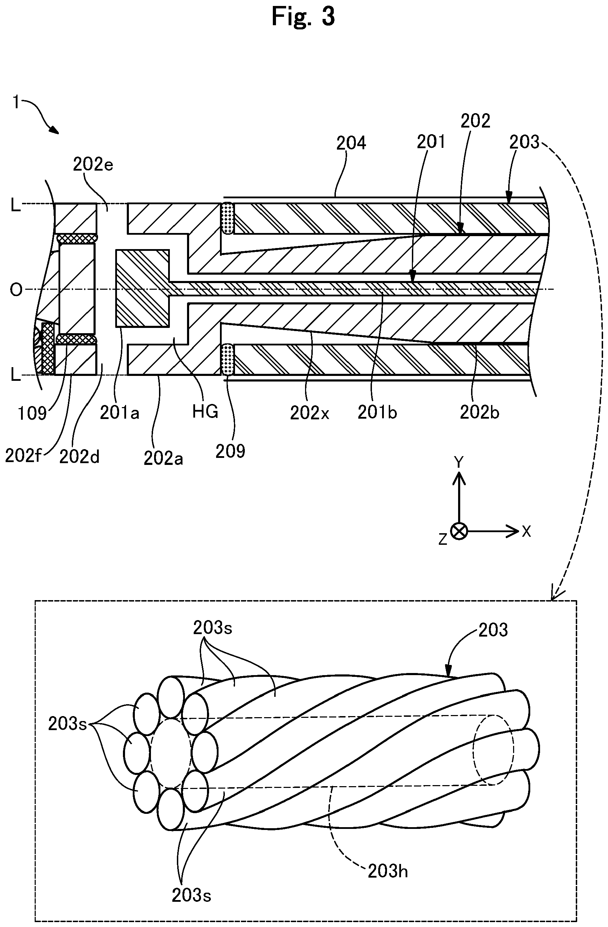

Fig. 2 shows an illustrative diagram of a cross-sectional configuration along the A-A line in Fig. 1. Fig. 3 shows an illustrative diagram of the configuration of the intermediate portion 200. As shown in Figs. 1 and 3, the intermediate portion 200 is disposed between the distal end portion 100 and the proximal end portion 300 of the guide wire 1, and includes a sensor 201, an accommodating portion 202, a hollow twisted wire 203, and a covering layer 204.

The sensor 201 is an optical pressure sensor for detecting the pressure of a body fluid flowing through the inside of a blood vessel, and disposed on the proximal end side of the distal end core 101 along the axis line O of the guide wire 1. The sensor 201 has a sensor head 201a disposed at the distal end portion thereof and a sensor cable 201b disposed at the proximal end side thereof. The sensor head 201a is a detection element (a microchip) for detecting blood pressure by a Fabry-Perot resonator which performs wavelength modulation of light in response to a change in an external pressure. It is noted that the sensor head 201a may be an optical detection element for detecting blood pressure by a means other than a Fabry-Perot resonator, or may be a non-optical detection element. The sensor cable 201b is an optical fiber for transmitting information detected with the sensor head 201a. The sensor cable 201b has a distal end portion connected to the sensor head 201a, and extends from the sensor head 201a toward the proximal end side of the guide wire 1, and has a proximal end portion connected to a controller (not shown) and a display (not shown). It is noted that the sensor 201 corresponds the "detection portion," and the sensor head 201a corresponds to the "detection element", and the sensor cable 201b corresponds to the "extension portion."

The accommodating portion 202 is a member for protecting the sensor 201 by accommodating the sensor 201 (the sensor head 201a and the sensor cable 201b) inside. The accommodating portion 202 has a housing portion 202a disposed at the distal end side thereof, a proximal end portion 202c disposed at the proximal end side thereof, and an intermediate portion 202b disposed between the housing portion 202a and the proximal end portion 202c.

The housing portion 202a is a member with a substantially closed-end cylindrical shape having the substantially same outer diameter as the second coil body 103. The sensor head 201a is housed in an inner space HG of the housing portion 202a. As indicated by broken lines in Fig. 2, through-holes (a first hole 202d and a second hole 202e) allowing communication between the inside and the outside of the housing portion 202a are formed on the side of the housing portion 202a at a part along the direction of the axis line O (the X-axis direction) and a part along the circumferential direction. During use of the guide wire 1, the first hole 202d and the second hole 202e each serve as a blood flow channel which allows blood in a blood vessel to flow into the inner space HG where the sensor head 201a is housed. Further, as shown in Figs. 1 and 3, a through-hole for insertion of the sensor cable 201b is formed on the bottom of the housing portion 202a. A housing distal end portion 202f having no communication hole is disposed at the distal end of the housing portion 202a. The inner surface of the housing distal end portion 202f is joined to the flange portion 101a of the distal end core 101 through a first joining region 109. The first joining region 109 can be formed by laser welding of the housing distal end portion 202f with the flange portion 101a. The first joining region 109 may be formed of any joining agent or adhesive.

The intermediate portion 202b is a member with a substantially cylindrical shape having a smaller outer diameter than the housing portion 202a. An inner cavity of the intermediate portion 202b is in communication with a through-hole at the bottom of the housing portion 202a, and accommodates the sensor cable 201b. A first tapered portion 202x having an outer diameter decreasing from the proximal end side to the distal end side thereof is disposed at the the distal end side of the intermediate portion 202b. A second tapered portion 202y having an outer diameter increasing from the proximal end side to the distal end side thereof is disposed at the the proximal end side of the intermediate portion 202b. The proximal end portion 202c is a member with a substantially cylindrical shape having a smaller outer diameter than the intermediate portion 202b. An inner cavity of the proximal end portion 202c is in communication with the inner cavity of the intermediate portion 202b, and accommodates the sensor cable 201b. The proximal end portion 202c including the housing portion 202a, the intermediate portion 202b, and the accommodating portion 202 is formed of a hyperelastic material, for example, a NiTi alloy, or an alloy of NiTi with an additional metal(s). It is noted that the housing portion 202a corresponds to the "first accommodating portion", and the intermediate portion 202b and the proximal end portion 202c correspond to the "second accommodating portion," and the first tapered portion 202x corresponds to the "diameter-decreasing portion."

The hollow twisted wire 203 is disposed so as to surround the intermediate portion 202b and the proximal end portion 202c in the circumferential direction of the guide wire 1, and covers the intermediate portion 202b, the proximal end portion 202c, and the distal end portion of a shaft 302. As shown in the lower panel of Fig. 3, the hollow twisted wire 203 in the present embodiment is a multi-thread coil configured such that a plurality of element wires 203s are wound to be multi-threaded. The hollow twisted wire 203 may be a single-thread coil configured such that a single element wire is wound to be single-threaded, or may be a single-thread twisted wire coil configured such that a plurality of twisted element wires are wound to be single-threaded, or may be a multi-thread twisted wire coil configured such that a plurality of twisted wires in which a plurality of element wires are twisted are each wound to be multi-thread. The intermediate portion 202b, the proximal end portion 202c, and the distal end portion of the shaft 302 are accommodated in an inner cavity 203h of the hollow twisted wire 203. The distal end portion of the hollow twisted wire 203 is connected (joined) to the proximal end portion of the housing portion 202a through a second joining region 209. The second joining region 209 can be formed of any joining agent, for example, a metal solder such as silver solder, gold solder, zinc, an Sn-Ag alloy, and an Au-Sn alloy; and an adhesive such as an epoxy-based adhesive. It is noted that the hollow twisted wire 203 corresponds to the "covering portion."

The hollow twisted wire 203 has the substantially same outer diameter at least at the distal end portion thereof (the maximum diameter of the outside of an element wire) as the proximal end portion of the housing portion 202a, and the hollow twisted wire 203 is preferably arranged side by side in the direction of the axis line O so as to be substantially coaxial with the housing portion 202a. In the examples as shown in Figs. 1 and 3, the hollow twisted wire 203 has the same outer diameter as the proximal end portion of the housing portion 202a throughout the entire region from the distal end side through the proximal end side thereof, and the hollow twisted wire 203 and the housing portion 202a are arranged side by side in the direction of the axis line O so as to be centered with respect with the axis line O. The hollow twisted wire 203 is formed of, for example, a material more plastically deformable than a hyperelastic material, for example, a stainless steel alloy such as SUS304 and SUS316.

The covering layer 204 is a hydrophilic coating layer covering the hollow twisted wire 203. In the example as shown in Fig. 1, the covering layer 204 covers each of the hollow twisted wire 203, the second joining region 209 disposed at the distal end portion of the hollow twisted wire 203, and a third joining region 309a disposed at the proximal end portion of the hollow twisted wire 203. The covering layer 204 can be formed of, for example, a hydrophilic resin such as urethane, polyimide, and nylon.

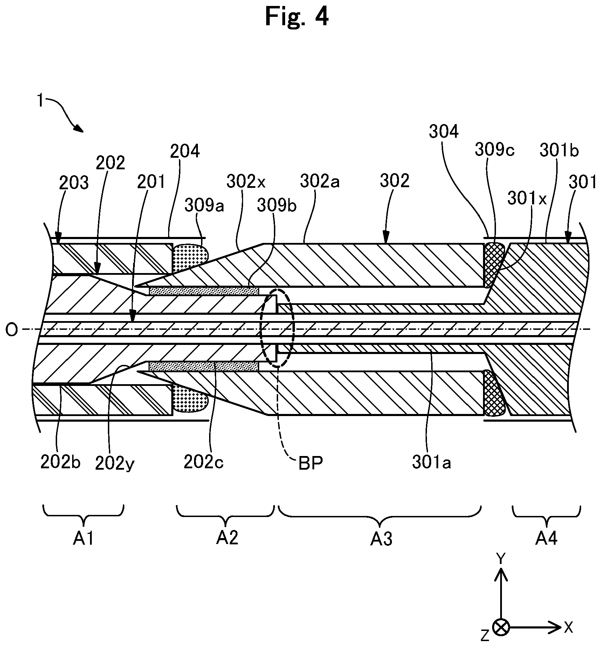

Fig. 4 shows an illustrative diagram of the configuration of the proximal portion 300. As shown in Figs. 1 and 4, the proximal end portion 300 is disposed at the proximal end side of the guide wire 1, and includes a hollow shaft 301, the shaft 302, and a proximal end side-covering layer 304.

As shown in Fig. 1, the hollow shaft 301 is a member for protecting the sensor cable 201b by accommodating the sensor cable 201b inside. The hollow shaft 301 has a distal end portion 301a disposed at the distal end side thereof, and a proximal end portion 301b continuing to the proximal end side.

The distal end portion 301a is a member with a substantially cylindrical shape having the substantially same bending stiffness as the proximal end portion 202c of the accommodating portion 202. As shown in Fig. 4, the hollow shaft 301 is connected to the shaft 302, and the shaft 302 is connected to the accommodating portion 202. That is, the hollow shaft 301 is connected to the accommodating portion 202 through the shaft 302. Connection between the hollow shaft 301 and the shaft 302, and between the shaft 302 and the accommodating portion 202 can be achieved by using any joining agent, for example, a metal solder such as silver solder, gold solder, zinc, an Sn-Ag alloy, and an Au-Sn alloy, and an adhesive such as an epoxy-based adhesive. In Fig. 4, the distal end portion of the distal end portion 301a and the proximal end portion of the proximal end portion 202c of the accommodating portion 202 are not connected to each other, but they may be connected. Hereinafter, the boundary portion between the distal end portion of the distal end portion 301a and the proximal end portion of the proximal end portion 202c may also be referred to as a "boundary portion BP" (Fig. 4). An inner cavity of the distal end portion 301a is in communication with the inner cavity of the proximal end portion 202c of the accommodating portion 202, and houses the sensor cable 201b. A tapered portion 301x having an outer diameter decreasing gradually from the proximal end side to the distal end side thereof is formed at the proximal end side of the distal end portion 301a.

The proximal end portion 301b is a member with a substantially cylindrical shape having the substantially same outer diameter as the shaft 302. An inner cavity of the proximal end portion 301b is in communication with the inner cavity of the distal end portion 301a, and houses the sensor cable 201b. The hollow shaft 301 including the distal end portion 301a, the tapered portion 301x, and the proximal end portion 301b is formed of a material more plastically deformable than a hyperelastic material, for example, a stainless steel alloy such as SUS304 and SUS316. It is noted that the hollow shaft 301 corresponds to the "shaft portion."

As shown in Fig. 4, the shaft 302 is a member covering the vicinity of the boundary portion BP between the accommodating portion 202 and the hollow shaft 301 to protect the boundary portion BP, and also enabling the stiffness of the guide wire 1 to be gradually changed. Specifically, the shaft 302 covers a part of the proximal end side of the accommodating portion 202 not covered with the hollow twisted wire 203 (that is, the proximal end portion 202c), the boundary portion BP between the accommodating portion 202 and the hollow shaft 301, and a small-diameter part of the distal end side of the hollow shaft 301 (that is, the distal end portion 301a). The shaft 302 has a tapered portion 302x formed at the distal end side thereof, and a proximal end portion 302a formed at the proximal end side thereof. The proximal end portion 302a is a member with a substantially cylindrical shape having the substantially same outer diameter as the proximal end portion 301b of the hollow shaft 301, and having a smaller inner diameter than the hollow twisted wire 203.

The tapered portion 302x is a portion having an outer diameter decreasing from the proximal end side to the distal end side thereof. As shown in Fig. 4, a part of the distal end side of the tapered portion 302x enters into the inside of the hollow twisted wire 203. The outer surface of the tapered portion 302x is joined to the proximal end portion of the hollow twisted wire 203 through the third joining region 309a. The inner surface of the proximal end portion 302a and the tapered portion 302x is joined to the outer surface of the proximal end portion 202c of the accommodating portion 202 through a fourth joining region 309b. Further, the proximal end portion 302a of the shaft 302 is joined to the tapered portion 301x of the hollow shaft 301 through a fifth joining region 309c. The third and fourth joining regions 309a and 309b can be formed of any joining agent, for example, a metal solder such as silver solder, gold solder, zinc, an Sn-Ag alloy, and an Au-Sn alloy; and an adhesive such as an epoxy-based adhesive. The third joining region 309a can be formed by laser welding of the hollow shaft 301 with the shaft 302. The third joining region 309a may be formed of any joining agent or adhesive. The shaft 302 including the tapered portion 302x and the proximal end portion 302a is formed of a material more plastically deformable than a hyperelastic material, for example, a stainless steel alloy such as SUS304 and SUS316. It is noted that the shaft 302 corresponds to the "proximal end side-covering portion."

Here, as shown in Fig. 4, a region where the accommodating portion 202 is covered with the hollow twisted wire 203, but not covered with the shaft 302 is referred to as a first region A1. Similarly, a region where the accommodating portion 202 is covered with the shaft 302, but not covered with the hollow twisted wire 203 is referred to as a second region A2. A region where the hollow shaft 301 is covered with the shaft 302 is referred to as a third region A3. A region where the hollow shaft 301 is not covered with the shaft 302 is referred to as a fourth region A4. Here, the stiffness of the second region A2 is equal to or larger than that of the first region A1. Further, the stiffness of the third region A3 is equal to or larger than that of the second region A2. Moreover, the stiffness of the fourth region A4 is equal to or larger than that of the third region A3. That is, the first to fourth regions have a stiffness gradually increasing from the distal end side toward the proximal end side thereof (stiffness: A1 ≤ A2 ≤ A3 ≤ A4). The difference in stiffness as described above is achieved by the different diameters of the accommodating portion 202 between the first and second regions A1 and A2, the different materials of the accommodating portion 202 and the hollow shaft 301 between the second and third regions A2 and A3, the different diameters of the shaft 302 between the second and third regions A2 and A3, and the different diameters of the hollow shaft 301 between the third and fourth regions A3 and A4.

The proximal end side-covering layer 304 is a fluorine-based resin coating layer covering an area from the fifth joining region 309c through the proximal end portion of the hollow shaft 301. The proximal end side-covering layer 304 can be formed of a fluorine-based resin, for example, PTFE, PFA, FEP, and the like.

In general, as shown in Fig. 1, the sensor cable 201b (the extension portion) for transmitting information detected with the sensor head 201a (the detection element) for detect information about a vessel such as a blood vessel has a smaller diameter than the sensor head 201a. The guide wire 1 according to the first embodiment includes the hollow twisted wire 203 (the covering portion) further covering the intermediate portion 202b and the proximal end portion 202c (the second accommodating portion) of the accommodating portion 202 accommodating the sensor cable 201b having a small diameter. In this configuration, the hollow twisted wire 203 can prevent scattering of the guide wire 1, and can also prevent a scattered piece from remaining in the body even if a breakage occurs at the intermediate portion 202b and the proximal end portion 202c of the accommodating portion 202. Further, the distal end portion of the above hollow twisted wire 203 is connected to the proximal end portion of the housing portion 202a (the first accommodating portion) of the accommodating portion 202 accommodating the sensor head 201a. This configuration can prevent scattering of the guide wire 1 at the boundary between the housing portion 202a and the intermediate portion 202b, and can also prevent a scattered piece from remaining in the body. Furthermore, the configuration where the distal end portion of the hollow twisted wire 203 is connected to the proximal end portion of the housing portion 202a can improve the torquability when the guide wire 1 is rotationally operated. These features of the guide wire 1 according to the first embodiment can reduce the risk of scattering for the the guide wire 1 capable of obtaining information about a vessel (blood pressure and coronary fractional flow reserve in the case of the first embodiment) in the inside of the vessel.

Further, as shown in Fig. 1, the distal end portion of the hollow twisted wire 203 (the covering portion) has the substantially same outer diameter as the proximal end portion of the housing portion 202a (the first accommodating portion), and the hollow twisted wire 203 is arranged side by side (side by side in the direction of the axis line O) so as to be substantially coaxial with the housing portion 202a (centered with respect to the axis line O). This configuration can allow an outer surface shape L (Fig. 3) at the connecting portion section between the distal end portion of the hollow twisted wire 203 and the proximal end portion of the housing portion 202a to be shaped to be flat without unevenness, preventing damages to tissues inside a blood vessel (a vessel).

Moreover, the configuration where the first tapered portion 202x (the diameter-decreasing portion) having an outer diameter gradually decreasing from a proximal end side to the distal end side thereof is formed at the the distal end portion of the intermediate portion 202b (the second accommodating portion) of the accommodating portion 202 as shown in Fig. 3 can allow the distal end portion of the intermediate portion 202b to be more flexible as compared with the proximal end side of the intermediate portion 202b.

Furthermore, as shown in Fig. 4, the stiffness of the region (the second region A2) of the intermediate portion 202b and the proximal end portion 202c (the second accommodating portion) of the accommodating portion 202 covered with the shaft 302 (the proximal end side-covering portion) but not covered with the hollow twisted wire 203 (the covering portion) is equal to or larger than the stiffness of the region (the first region A1) of the intermediate portion 202b and the proximal end portion 202c of the accommodating portion 202 covered with the hollow twisted wire 203 but not covered with with the shaft 302. Further, the stiffness of the region (the third region A3) of the hollow shaft 301 (the shaft portion) covered with the shaft 302 is equal to or larger than that of the second region A2. Moreover, the stiffness of the region (the fourth region A4) of the hollow shaft 301 (the shaft portion) not covered with the shaft 302 is equal to or larger than that of the third region A3. The above configurations enable the stiffness of the guide wire to be gradually decreased from the proximal end side to the distal end side thereof, and can provide a guide wire excellent in supporting capability, torquability, and vascular selectivity. Furthermore, the configuration where the shaft 302 covers the boundary portion BP between the accommodating portion 202 and the hollow shaft 301 and the both ends thereof (that is, a part of the proximal end side of the accommodating portion 202 not covered with the hollow twisted wire 203, and a part of the distal end side of the hollow shaft 301) can prevent occurrence of a breakage and/or a kink at the boundary portion BP between the accommodating portion 202 and the hollow shaft 301, improving the durability of the guide wire 1.

Further, the fracture durability at the housing portion 202a (the first accommodating portion) and the intermediate portion 202b and the proximal end portion 202c of the accommodating portion 202 (the second accommodating portion) can be improved, for example, by using a hyperelastic material having high fatigue strength such as a NiTi alloy and an alloy of NiTi with an additional metal(s). Moreover, when a material more plastically deformable than a hyperelastic material, for example, a stainless steel alloy such as SUS304 and SUS316 is used for the hollow twisted wire 203 (the covering portion), the shaft 302 (the proximal end side-covering portion), and the hollow shaft 301 (the shaft portion), the guide wire 1 can have a stiffness gradually decreasing from the proximal end side to the distal end side thereof, thereby providing the guide wire 1 excellent in supporting capability, torquability, and vascular selectivity.

Moreover, in the configuration where the shaft 302 (the covering portion) is a coil body configured such that one or more element wires are wound spirally, breakage at both the shaft 302 and the intermediate portion 202b and the proximal end portion 202c (the second accommodating portion) of the accommodating portion 202, if occurs, would merely cause the element wire(s) of the shaft 302 to unwind and extend. This can further prevent scattering of the guide wire, and can further prevent a scattered piece from remaining in the body.

Furthermore, in the configuration where the sensor head 201a (the detection element) is for detecting the pressure of a body fluid flowing through the inside of a vessel such as a blood vessel, the guide wire 1 can serve as a device for detecting (measuring) blood pressure. Further, the presence of the distal end coil 110 disposed on the distal end side of the housing portion 202a (the first accommodating portion) and configured such that one or more element wires are wound spirally can improve the flexibility at the distal end side, and thus can reduce the risk of damages on tissues inside a vessel.

### Second embodiment

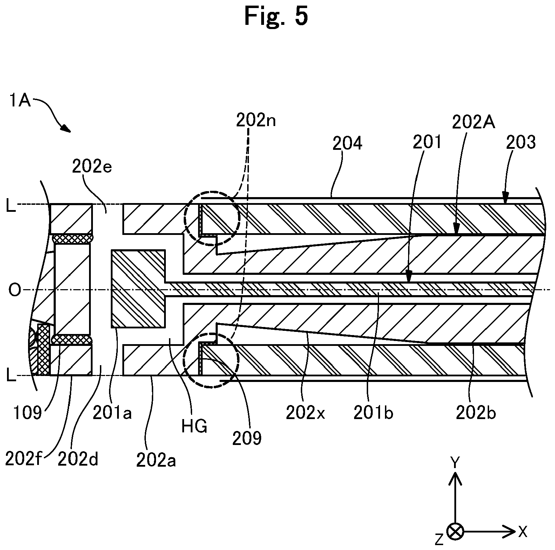

Fig. 5 shows an illustrative diagram of the configuration of the intermediate portion 200 of a guide wire 1A according to a second embodiment. The guide wire 1A according to the second embodiment includes an accommodating portion 202A at the intermediate portion 200 in place of the accommodating portion 202. For the accommodating portion 202A, a stepped portion 202n for engaging with the distal end portion of the hollow twisted wire 203 is formed at the proximal end portion of the housing portion 202a. The stepped portion 202n is a notch formed at the bottom corner of the housing portion 202a, and extends in the circumferential direction of the guide wire 1A. The hollow twisted wire 203 is joined to the second joining region 209 in a state where the distal end portion of the hollow twisted wire 203 is in engagement with the stepped portion 202n of the accommodating portion 202A.

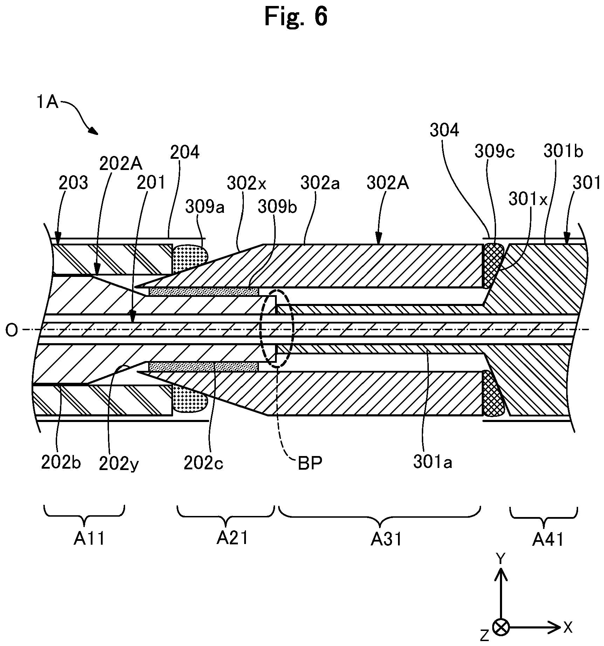

Fig. 6 shows an illustrative diagram of the configuration of the proximal portion 300 of the guide wire 1A according to the second embodiment. The guide wire 1A according to the second embodiment includes a shaft 302A at the proximal end portion 300 in place of the shaft 302. The shaft 302A is formed of a material different from the first embodiment, specifically, formed of a hyperelastic material, for example, a NiTi alloy or an alloy of NiTi with an additional metal(s). Here, as shown in Fig. 6, a region where the accommodating portion 202A is covered with the hollow twisted wire 203, but not covered with the shaft 302A is referred to as a first region A11. Similarly, a region where the accommodating portion 202A is covered with the shaft 302A, but not covered with the hollow twisted wire 203 is referred to as a second region A21. A region where the hollow shaft 301 is covered with the shaft 302A is referred to as a third region A31. A region where the hollow shaft 301 is not covered with the shaft 302A is referred to as a fourth region A41. Here, the stiffness of the second region A21 is equal to or larger than that of the first region A11 as in the first embodiment. The stiffness of the third region A31 is equal to or larger than that of the second region A21. The stiffness of the fourth region A41 is equal to or larger than that of the third region A31. (stiffness: A11 ≤ A21 ≤ A31 ≤ A41).

The shape of the housing portion 202a of the accommodating portion 202A may optionally be changed as described above. For example, the stepped portion 202n for engaging with the hollow twisted wire 203 may be formed. Alternatively, the housing portion 202a may be configured, for example, to have a shape other than the substantially closed-end cylindrical shape (for example, a spherical shape having an inner space HG and a through-hole serving as a blood flow channel). Further, the material of the shaft 302A may optionally be changed. For example, the aforementioned hyperelastic materials or resin materials may be used. These configurations can also produce similar effects as in the first embodiment. Moreover, the configuration of the guide wire 1A according to the second embodiment where the stepped portion 202n engaging with the distal end portion of the hollow twisted wire 203 (the covering portion) is formed at the proximal end portion of the housing portion 202a (the first accommodating portion) enables easy positioning of the housing portion 202a with the hollow twisted wire 203 at the time of manufacture. Moreover, locally increased stiffness can be avoided at the connection section between the housing portion 202a and the hollow twisted wire 203 (that is, a portion in which the second joining region 209 is disposed) as compared with the configuration described in the the first embodiment where the stepped portion 202n is not disposed.

Further, the guide wire 1A according to the second embodiment where the shaft 302A (the proximal end side-covering portion) is made of a hyperelastic material enables the stiffness to be gradually changed over the first to fourth regions A11 to A41. Moreover, the fracture durability at the housing portion 202a (the first accommodating portion) and the intermediate portion 202b and the proximal end portion 202c (the second accommodating portion) of the accommodating portion 202A can be improved, for example, by using a hyperelastic material having high fatigue strength such as a NiTi alloy and an alloy of NiTi with an additional metal(s). Furthermore, use of a material more plastically deformable than a hyperelastic material, for example, a stainless steel alloy such as SUS304 and SUS316 for the hollow twisted wire 203 (the covering portion) and the hollow shaft 301 (the shaft portion) enables the stiffness of the guide wire 1A to be gradually decreased from the proximal end side to the distal end side thereof, thereby providing the guide wire 1A excellent in supporting capability, torquability, and vascular selectivity.

### Third embodiment

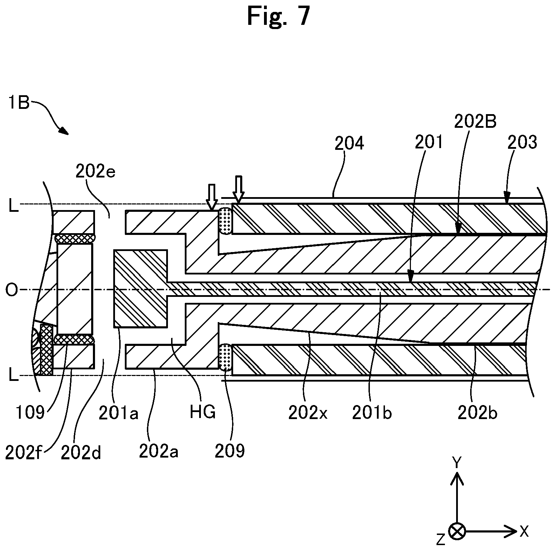

Fig. 7 shows an illustrative diagram of the configuration of the intermediate portion 200 of a guide wire 1B according to a third embodiment. The guide wire 1B according to the third embodiment includes an accommodating portion 202B at the intermediate portion 200 in place of the accommodating portion 202. The housing portion 202a of the accommodating portion 202B has an outer diameter smaller than that of the hollow twisted wire 203. This creates a step (white arrow heads in Fig. 7) at an outer surface shape L of the guide wire in the connection section between the distal end portion of the hollow twisted wire 203 and the proximal end portion of the housing portion 202a of the guide wire 1B. As described above, the outer diameter of each constituent member of the guide wire 1B (for example, the distal end coil 110, the accommodating portion 202B, the hollow twisted wire 203, the hollow shaft 301, and the shaft 302) can optionally be changed. These configurations can also produce similar effects as in the first embodiment.

### Fourth embodiment

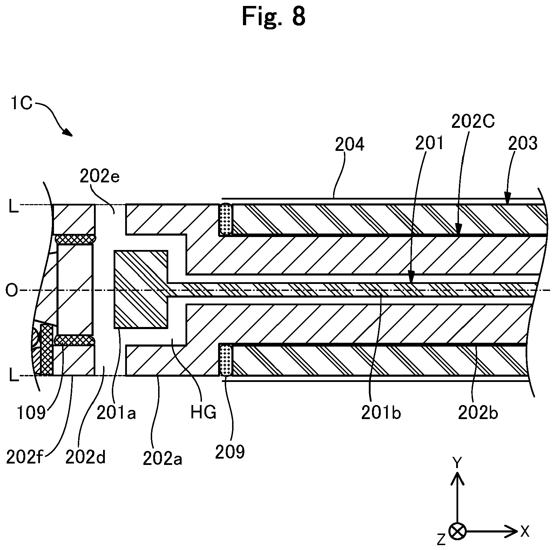

Fig. 8 shows an illustrative diagram of the configuration of the intermediate portion 200 of a guide wire 1C according to a fourth embodiment. The guide wire 1C according to the fourth embodiment includes an accommodating portion 202C at the intermediate portion 200 in place of the accommodating portion 202. In the accommodating portion 202C, the diameter-decreasing portion (the first tapered portion 202x in Fig. 3) is not formed at the distal end of the intermediate portion 202b. As described above, the diameter-decreasing portions and the diameter-increasing portions in each constituent member of the guide wire 1C (for example, the first tapered portion 202x and the second tapered portion 202y of the accommodating portion 202C, the tapered portion 101c of the distal end core 101, the tapered portion 302x of the shaft 302, the tapered portion 301x of the hollow shaft 301, and the like) may be omitted. These configurations can also produce similar effects as in the first embodiment.

### Fifth embodiment

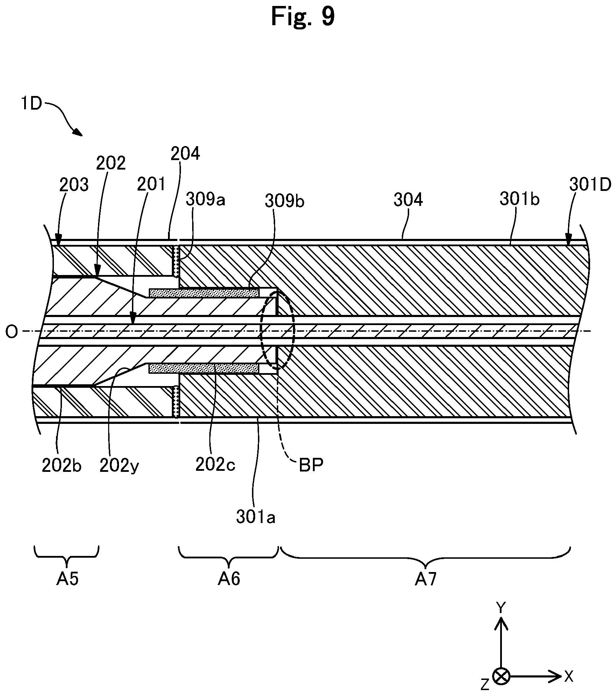

Fig. 9 shows an illustrative diagram of the configuration of the proximal portion 300 of a guide wire 1D according to a fifth embodiment. In the proximal end portion 300, the guide wire 1D according to the fifth embodiment includes neither the shaft 302 (Fig. 4) nor the fifth joining region 309c (Fig. 4), but includes a hollow shaft 301D in place of the hollow shaft 301. The hollow shaft 301D has the substantially same outer diameter as the hollow twisted wire 203, and has a distal end portion 301a in which a stepped portion for fitting the proximal end portion 202c of the accommodating portion 202 at the internal surface thereof, and the proximal end portion 301b continuously extending to the proximal end side of the distal end portion 301a. The proximal end portion 202c of the accommodating portion 202 in a state where it is fitted into the stepped portion of the distal end portion 301a is joined through the fourth joining region 309b and the boundary portion BP.

Here, as shown in Fig. 9, a region where the accommodating portion 202 is covered with the hollow twisted wire 203 is referred to as a fifth region A5. Further, a region where the accommodating portion 202 is covered with the hollow shaft 301D is referred to as a sixth region A6. Moreover, a region where the accommodating portion 202 is not covered with the hollow shaft 301D is referred to as a seventh region A7. Here, the stiffness of the sixth region is equal to or larger than that of the fifth region. The stiffness of the seventh region is equal to or larger than that of the sixth region (stiffness: A5 ≤ A6 ≤ A7). Such difference in stiffness is brought by the difference in diameters of the accommodating portion 202 over the fifth to seventh regions A5 to A7 and the difference in diameters of the hollow shaft 301D.

As described above, the configuration of the proximal end portion 300 can optionally be changed. For example, the shaft 302 may be omitted, or a plurality of shafts 302 may be included, or the configuration of the hollow shaft 301D may be changed. These configurations can also produce similar effects as in the first embodiment.

### Sixth embodiment

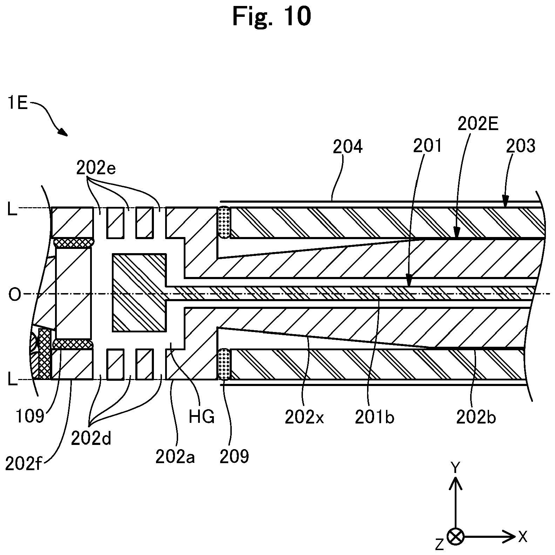

Fig. 10 shows an illustrative diagram of the configuration of the intermediate portion 200 of a guide wire 1E according to a sixth embodiment. The guide wire 1E according to the sixth embodiment includes an accommodating portion 202E at the intermediate portion 200 in place of the accommodating portion 202. In the accommodating portion 202E, a plurality of sets (3 sets in the example of the figure) of the first holes 202d and the second holes 202e are formed at the housing portion 202a. As described above, the shape of the housing portion 202a of the accommodating portion 202A can optionally be changed. For example, a plurality of sets of the first holes 202d and the second holes 202e may be formed. Further, for example, either one of the first hole 202d and the second hole 202e may be omitted to have a congratulation where a single through-hole is formed, or the housing portion 202a may be configured to be a porous body. These configurations can also produce similar effects as in the first embodiment. Further, the guide wire 1E according to the sixth embodiment can promote an easier flow of intravascular blood into the inner space HG where the sensor head 201a is housed.

### Seventh embodiment

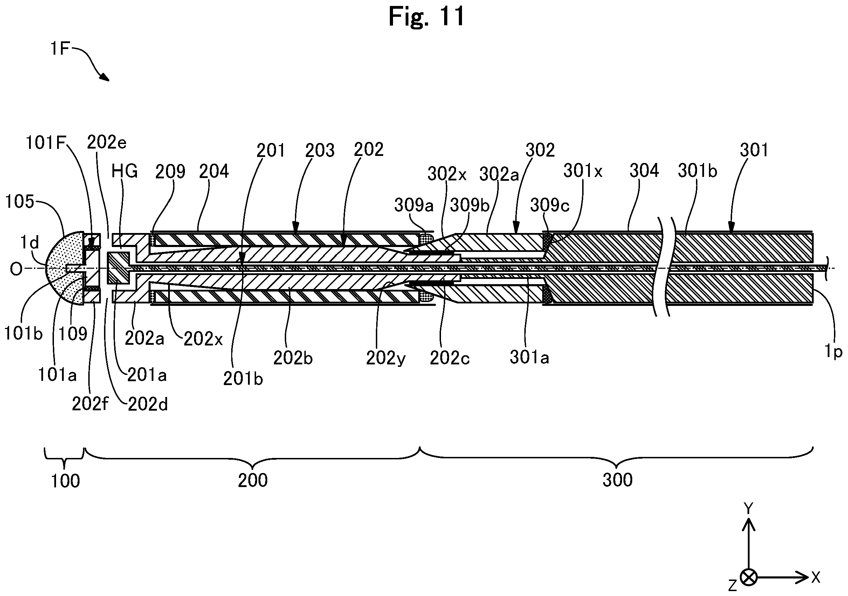

Fig. 11 shows an illustrative diagram of a cross-sectional configuration of a guide wire 1F according to a seventh embodiment. The guide wire 1F according to the seventh embodiment does not include the distal end coil 110 (Fig. 1), but includes a distal end core 101F at the distal end portion 100 in place of the distal end core 101. In the distal end core 101F, the tapered portion 101c (Fig. 1) is not disposed at the distal end portion of the flange portion 101a, but the distal end portion 101b is directly disposed. Further, the proximal end portion of the distal end tip 105 is joined to the housing distal end portion 202f of the accommodating portion 202. As described above, the configuration of the distal end portion 100 can optionally be changed. For example, the distal end coil 110 may be omitted, or the shape of the distal end core 101F may be changed, or the distal end tip 105 may be omitted. These configurations can also produce similar effects as in the first embodiment.

### Eighth embodiment

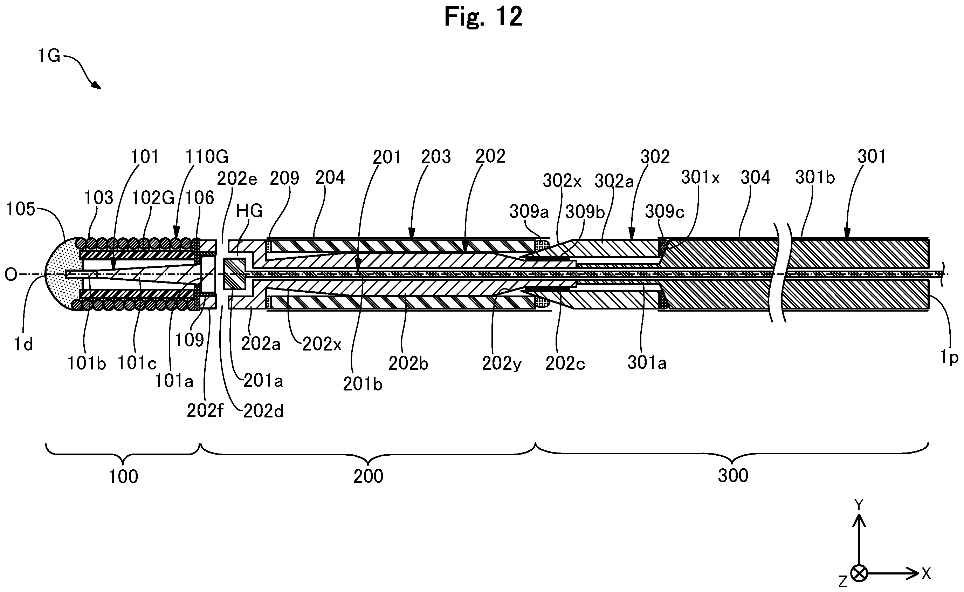

Fig. 12 shows an illustrative diagram of a cross-sectional configuration of a guide wire 1G according to an eighth embodiment. The guide wire 1G according to the eighth embodiment includes a distal end coil 110G at the distal end portion 100 in place of the distal end coil 110. The distal end coil 110G includes a first tubular body 102G of a substantially cylindrical tube in place of the first coil body 102 (Fig. 1) as a coil body. The first tubular body 102G may be formed of a similar material as the first coil body 102, or may be formed of a resin material. As described above, the configuration of the distal end portion 100 can optionally be changed. For example, at least one of the first coil body 102 and the second coil body 103 (Fig. 1) may be configured as a tubular body, or at least one of the first coil body 102 and the second coil body 103 may be omitted. These configurations can also produce similar effects as in the first embodiment.

### Ninth embodiment

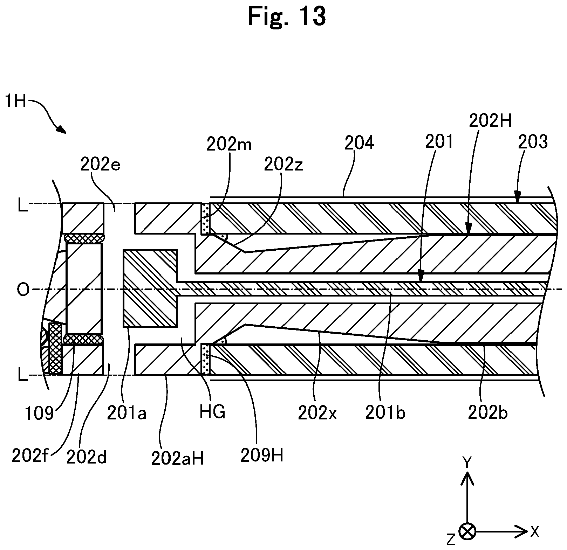

Fig. 13 shows an illustrative diagram of the configuration of the intermediate portion 200 of a guide wire 1H according to a ninth embodiment. The guide wire 1H according to the ninth embodiment includes an accommodating portion 202H at the intermediate portion 200 in place of the accommodating portion 202. The accommodating portion 202H include a housing portion 202aH in which a tapered portion 202z and a stepped portion 202m are formed. The tapered portion 202z has an inclination formed at the bottom of the housing portion 202aH, and has an outer diameter gradually increasing from the proximal end side to the distal end side thereof. The stepped portion 202m corresponds to a notch formed at the tapered portion 202z. In the example as shown in Fig. 13, the hollow twisted wire 203 is joined through a second joining region 209H in a state where the distal end portion thereof is engaged with the stepped portion 202m.

As described above, the shape of the housing portion 202aH can optionally be changed. The tapered portion 202z having an outer diameter gradually increasing from the proximal end side to the distal end side thereof may be disposed at the proximal end portion of the housing portion 202aH. This configuration can also produce similar effects as in the first embodiment. Further, the configuration according to the ninth embodiment where the tapered portion 202z is disposed at the proximal end portion of the housing portion 202aH can relieve stress concentration occurring between the housing portion 202aH and the first tapered portion 202x. This in turn can improve fatigue strength of the accommodating portion 202H, improving durability against fracture due to bending and twisting.

### Variations of present embodiments

The present invention shall not be limited to the above embodiments, but can be implemented according to various aspects without departing from the scope and spirit of the present invention. For example, the following variations may be possible.

### Variation 1

In the above first to ninth embodiments, the configurations of the guide wires 1, and 1A to 1H are exemplified. However, various changes may be made in the configuration of the guide wire 1. For example, each of the guide wires according to the above embodiments is described as a device which can be inserted into a blood vessel and used to detect blood pressure. However, the guide wire 1 may be configured as a device which can be inserted into a vessel such as the vascular system and the lymph gland system, and used to detect/obtain information (temperature, pressure, an image, and the like) about the vessel. Further, the guide wire 1 may be configured as a device which can be inserted into an organ in the human body such as the vascular system, the lymph gland system, the biliary tract system, the urinary tract system, the respiratory tract system, the digestive organ system, secretory glands, reproductive organs, and the like, and used to detect/obtain information (temperature, pressure, an image, and the like) about a body lumen.

### Variation 2

In the above first to ninth embodiments, the configurations of the intermediate portions 200 are exemplified. However, various changes may be made in the configurations of the intermediate portions 200. For example, the hollow twisted wire 203 may be configured as a substantially cylindrical tube (a tubular body) instead of a coil body. For example, at least one of the outer diameter and the inner diameter of the hollow twisted wire 203 may not necessarily be constant. For example, the proximal end portion of the hollow twisted wire 203 may be connected to the distal end portion of the hollow shaft 301. In this case, the shaft 302 may be omitted, or the shaft 302 may be arranged inside the hollow twisted wire 203 (which corresponds to a configuration where the shaft 302 is covered with the hollow twisted wire 203). For example, the covering layer 204 may be omitted, or the covering layer 204 may be configured so as to cover each portion of the distal end portion 100. For example, at least one of the accommodating portion 202 and the hollow twisted wire 203 may be formed of a material (for example, a resin material) other than those listed above.

### Variation 3

In the above first to ninth embodiments, the configurations of the proximal portions 300 are exemplified. However, various changes may be made in the configurations of the intermediate portions 300. For example, the hollow shaft 301 and the shaft 302 may be formed integrally. For example, the hollow shaft 301 may be composed of a core shaft and a coil body covering the core shaft. For example, the hollow shaft 301 and the shaft 302 may be omitted, and the accommodating portion 202 and the hollow twisted wire 203 may extend to the proximal end portion of the guide wire 1. For example, the stiffness of the first regions A1 and A11, the second regions A2 and A21, the third regions A3 and A31, and the fourth regions A4 and A41 may not necessarily follow the relationship of stiffness of A1 ≤ A2 ≤ A3 ≤ A4.

For example, the proximal end side-covering layer 304 may be omitted, or the proximal end side-covering layer 304 and the covering layer 204 may be formed integrally. For example, the proximal end side-covering layer 304 and the covering layer 204 may be formed in layers (stacked in the circumferential direction of the guide wire 1). For example, at least one of the hollow shaft 301 and the shaft 302 may be formed of a material (for example, a resin material) other than those listed above.

### Variation 4

The configurations of the guide wires 1 and 1A to 1H according to the first to ninth embodiments, and the configurations of the guide wires 1 and 1A to 1H according to the variations 1 to 3 may be combined in an appropriate manner. For example, the configurations of the distal end portions 100 described in any of the ninth and eighth embodiments, the configurations of the intermediate portions 200 described in the second, third, fourth, sixth, and ninth embodiments, the configurations of the proximal end portions 300 described in the second and fifth embodiments can each be combined in any fashion.

As described above, the present aspect is described based on the embodiments and variations, but the aforementioned modes of implementing the aspect are provided merely for better understanding of the present aspect, and shall not be construed as limiting the present aspect. Alternations and improvements may be made in the present aspect without departing from the scope and spirit of the claims, and equivalents thereof fall within the scope of the present aspect. Moreover, a technical feature may be omitted, if desired, unless otherwise stated as essential in the present specification.

### DESCRIPTION OF THE REFERENCE NUMERALS

- 1, 1A to 1H: Guide wire
- 100: Distal end portion
- 101,: 101F Distal end core
- 101a: Flange portion
- 101b: Distal end portion
- 101c: Tapered portion
- 102, 102G: First coil (tubular) body
- 103: Second coil body
- 105: Distal end tip
- 106: Joining region
- 109: First joining region
- 110, 110G: Distal end core
- 200: Intermediate portion
- 201: Sensor
- 201a: Sensor head
- 201b: Sensor cable
- 202, 202A, 202B, 202C, 202E: Accommodating portion
- 202a, 202aH: Housing portion
- 202b: Intermediate portion
- 202c: Proximal end portion
- 202d: First hole
- 202e: Second hole
- 202f: Housing distal end portion
- 202n, 202m: Stepped portion
- 202x: First tapered portion
- 202y: Second tapered portion
- 202z: Tapered portion
- 203: Hollow twisted wire
- 204: Covering layer
- 209: Second joining region
- 300: Proximal portion
- 301, 301D: Hollow shaft
- 301a: Distal end portion
- 301b: Proximal end portion
- 301x: Tapered portion
- 302,302A: Shaft
- 302a: Proximal end portion
- 302x: Tapered portion
- 304: Proximal end side-covering layer
- 309a: Third joining region
- 309b: Fourth joining region
- 309c: Fifth joining region

## Claims

1. A guide wire, comprising:
a detection portion including a detection element for detecting information about a vessel in an inside of the vessel, and an extension portion for transmitting the information detected with the detection element, the extension portion extending from the detection element toward a proximal end side of the guide wire;
an accommodating portion having a first accommodating portion accommodating the detection element and a second accommodating portion accommodating the extension portion; and
a covering portion covering the second accommodating portion, wherein
the covering portion having a distal end portion connected to a proximal end portion of the first accommodating portion.

2. The guide wire according to claim 1, wherein
the covering portion
has an outer diameter at the distal end portion substantially equal to an outer diameter of the proximal end portion of the first accommodating portion, and
is arranged side by side coaxial with the first accommodating portion.

3. The guide wire according to claim 1 or 2, wherein
a diameter-decreasing portion having an outer diameter gradually decreasing from a proximal end side to a distal end side is formed at a distal end side of the second accommodating portion.

4. The guide wire according to claim 3, wherein
a stepped portion engaging with the distal end portion of the covering portion is formed at the proximal end portion of the first accommodating portion.

5. The guide wire according to any one of claims 1 to 4, further comprising:
a shaft portion disposed on a proximal end side of the second accommodating portion, and
a proximal end side-covering portion covering a part of the proximal end side of the second accommodating portion not covered with the covering portion, a boundary portion between the second accommodating portion and the shaft portion, and a part of a distal end side of the shaft portion,
wherein
a second region has a stiffness equal to or larger than a stiffness of a first region,
the first region being a region of the second accommodating portion covered with the covering portion but not covered with the proximal end side-covering portion, and
the second region being a region of the second accommodating portion not covered with the covering portion but covered with the proximal end side-covering portion.

6. The guide wire according to claim 5, wherein
a third region has a stiffness equal to or larger than a stiffness of the second region,
the third region being a region of the shaft portion covered with the proximal end side-covering portion.

7. The guide wire according to claim 6, wherein
a fourth region has a stiffness equal to or larger than a stiffness of the third region,
the fourth region being a region of the shaft portion not covered with the proximal end side-covering portion.

8. The guide wire according to any one of claims 5 to 7, wherein
the first accommodating portion, the second accommodating portion, and the proximal end side-covering portion are formed of a hyperelastic material, and
the covering portion and the shaft portion are formed of a material more plastically deformable than the hyperelastic material.

9. The guide wire according to any one of claims 5 to 7, wherein
the first accommodating portion and the second accommodating portion are formed of a hyperelastic material, and
the covering portion, the proximal end side-covering portion, and the shaft portion are formed of a material more plastically deformable than the hyperelastic material.

10. The guide wire according to any one of claims 1 to 9, wherein
the covering portion is a coil body configured such that one or more element wires are wound spirally.

11. The guide wire according to any one of claims 1 to 10, wherein
the detection element is for detecting a pressure of a body fluid flowing through the inside of the vessel.

12. The guide wire according to any one of claims 1 to 11, further comprising:
a distal end coil disposed on a distal end side of the first accommodating portion, the distal end coil being configured such that one or more element wires are wound spirally.
